# EUROPÄISCHE PATENTSCHRIFT

(11) **EP 2 697 184 B1**
(45) Veröffentlichungstag und Bekanntmachung des Hinweises auf die Patenterteilung: **23.03.2016**
(21) Anmeldenummer: 12723086.0
(22) Anmeldetag: 03.04.2012
(51) Int. Cl.: C07B 43/04

(54) **VERFAHREN ZUR HERSTELLUNG VON AROMATISCHEN UND HETEROAROMATISCHEN AMINEN**
PROCESS FOR PREPARING AROMATIC AND HETEROAROMATIC AMINES
PROCÉDÉ DE PRODUCTION D'AMINES AROMATIQUES ET HÉTÉROAROMATIQUES

(30) Priorität: 14.04.2011 DE 102011017027
(43) Veröffentlichungstag der Anmeldung: 19.02.2014
(73) Patentinhaber: Studiengesellschaft Kohle mbH, 45470 Mülheim an der Ruhr (DE)
(72) Erfinder: REETZ, Manfred, 35043 Marburg (DE); MEHLER, Gerlinde, 45470 Mülheim an der Ruhr (DE)
(86) Internationale Anmeldenummer: PCT/DE2012/100089
(87) Internationale Veröffentlichungsnummer: WO 2012/139561

(56) Entgegenhaltungen:
- EP-A1- 0 382 258
- US-A- 6 124 462
- US-A1- 2004 147 392
- ACKERMANN LUTZ; SPATZ JULIA H; GSCHREI CHRISTIAN J; BORN ROBERT; ALTHAMMER ANDREAS: "A diaminochlorophosphine for palladium-catalyzed arylations of amines and ketones.", ANGEWANDTE CHEMIE (INTERNATIONAL ED. IN ENGLISH), Bd. 45, Nr. 45, 2006, Seiten 7627-7630, XP002680604, in der Anmeldung erwähnt
- AHMADIBENI YOUSEF; PARANG KEYKAVOUS: "Solid-phase synthesis of symmetrical 5 ',5 '-dinucleoside mono-, di-, tri-, and tetraphosphodiesters", ORGANIC LETTERS, Bd. 9, Nr. 22, 2007, Seiten 4483-4486, XP002680605,
- KITAS E A; KNORR R; TRZECIAK A; BANNWARTH W: "ALTERNATIVE STRATEGIES FOR THE FMOC SOLID-PHASE SYNTHESIS OF O-4 PHOSPHO-L-TYROSINE-CONTAINING PEPTIDES", HELVETICA CHIMICA ACTA, Bd. 74, 1991, Seiten 1314-1328, XP002680606,
- SLOT VAN DER S C ET AL: "RHODIUM-CATALYZED HYDROFORMYLATION AND DEUTERIOFORMYLATION WITH PYRROLYL-BASED PHOSPHORUS AMIDITE LIGANDS: INFLUENCE OF ELECTRONIC LIGAND PROPERTIES", ORGANOMETALLICS, ACS, WASHINGTON, DC, US, vol. 21, no. 19, 16 September 2002 (2002-09-16), pages 3873-3883, XP001126452, ISSN: 0276-7333, DOI: 10.1021/OM010760Y

## Beschreibung

Die vorliegende Erfindung betrifft ein Verfahren zur Herstellung von aromatischen und heteroaromatischen Aminen aus den entsprechenden Aryl- und Heteroarylhalogeniden oder Sulfonaten in Gegenwart eines Katalysators und einer Base.

Aromatische und heteroaromatische Amine sind von großem industriellem Interesse. Die Herstellung dieser Verbindungen über die katalytische Aminierung der entsprechenden Chlor-, Brom- oder lodverbindungen unter Bildung von Arylaminen ist eine in der organischen Synthese bedeutsame Stoffumwandlung (B. Schlummer, U. Scholz, Adv. Synth. Catal. 2004, 346, 1599). In der Regel benötigt die Reaktion sowohl eine Base zum Binden der freigesetzten Säure HX (X = Cl, Br oder I), als auch einen Übergangsmetallkatalysator. Unter den verwendeten Übergangsmetallen befinden sich Palladium, Nickel, Eisen und Kobalt. Es hat sich dabei herausgestellt, dass Palladium (Pd) das wirksamste Übergangsmetall ist. Dazu sind Liganden zur Stabilisierung und Aktivierung des Metalls erforderlich, welches in der Oxidationsstufe Null (Pd⁰) die Reaktion durch reduktive Einschiebung in die Kohlenstoff-Halogen-Bindung initiiert. Leider musste man feststellen, dass leicht zugängliche bzw. billige Phosphane wie z. B. Triphenylphosphan nicht gut geeignet sind, denn deren Verwendung führt zu niedrigen bzw. unbrauchbaren Ausbeuten. Als Durchbruch auf diesem Forschungsgebiet gelten insbesondere die Entdeckungen von Buchwald *(*Acc. Chem. Res. 1998, 31, 805; J. Org. Chem. 2000, 65, 5327; WO 2006/074315 A2; neuere Übersicht: Angew. Chem. Int. Ed. 2008, 47, 6338), von Hartwig *(*J. Am. Chem. Soc. 1998, 120, 7369*;* J. Am. Chem. Soc. 2006, 128, 10028; J. Am. Chem. Soc. 2008, 130, 6586) und von anderen Autoren (Zusammenfassung: A. F. Littke und G. C. Fu, Angew. Chem. Int. Ed. 2002, 41, 4176). Danach katalysieren Pd-Komplexe von sperrigen Phosphanen wie z. B. **1-8** die Pdbedingte Aminierung am besten, denn Ausbeuten von mehr als 80 % können für viele Arylhalogenide bzw. unterschiedliche Amin-Ausgangsverbindungen erzielt werden.

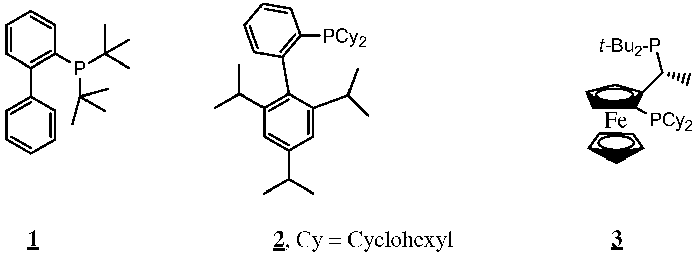

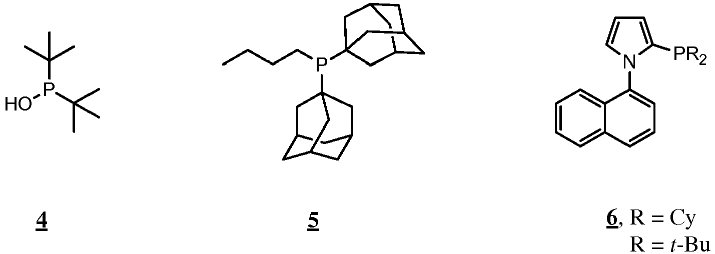

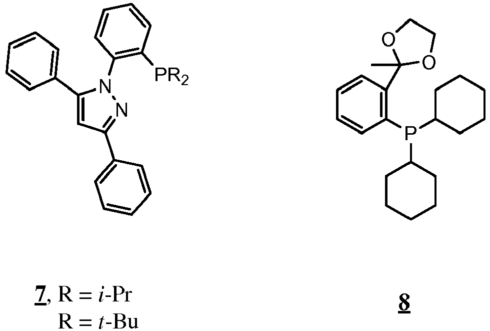

Der deutliche Nachteil der obigen Methoden, die als "state of the art" gelten, ist die Tatsache, dass sperrige Liganden des obigen Typs keineswegs leicht zugänglich sind, vielmehr erfordern ihre Synthesen nicht nur teure Organolithium- oder Organomagnesium-Reagenzien, sondern mehrere Stufen unter Schutzgas-Bedingungen und bei tiefen Temperaturen Cyclische Phosphorverbindungen des Typs **9-11** wurden ebenfalls als Liganden bei Pd-katalysierten Aminierungen von Arylhalogeniden eingesetzt, die Synthese der entsprechenden sperrigen Diamine erfordert jedoch ebenfalls eine aufwändige mehrstufige Reaktionssequenz (L. Ackermann, et al, Angew. Chem. Int. Ed. 2006, 45, 7627; J. C. Verkade, et al, J. Org. Chem. 2003, 68 8416). Auch weitere sperrige P-Liganden sind zwar für Pd-katalysierte Aminierungen geeignet, erfordern jedoch wiederum aufwändige Synthesen (G. Y. Li, Angew. Chem. Int. Ed. 2001, 40, 1513; US Patent 20040147392, 2004; B. Schlummer, U. Scholz, Adv. Synth. Catal. 2004, 346, 1599; M. Beller, et al, Chem. Eur. J. 2004, 10, 2983; J. A. Coggan, et al, US Patent 7,563,932, 2009; R. A. Singer, et al, Tetrahedron Lett. 2006, 47, 3727).

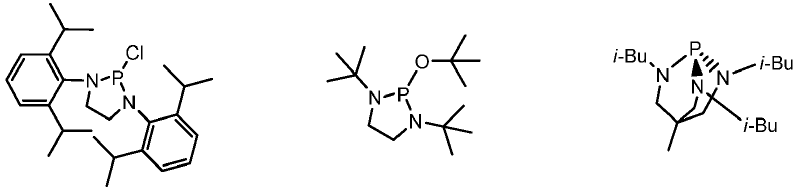

Die aus dem Stand der Technik bekannten Verfahren benötigen zur Herstellung der aromatischen Amine teure und nur über aufwändige Syntheseverfahren zugängliche Phosphorverbindungen. Der vorliegenden Erfindung lag somit die Aufgabe zugrunde, ein Verfahren zur Verfügung zu stellen, in welchem aromatische und heteroaromatische Aminverbindungen auf einfache Weise unter Verwendung kostengünstiger Katalysatoren hergestellt werden können.

Gegenstand der vorliegenden Erfindung ist demgemäß ein Verfahren zur Herstellung von aromatischen und heteroaromatischen Aminen der allgemeinen Formel I

Ar-NR¹R² (I)

worin
Ar für einen substituierten oder unsubstituierten Aryl-, Heteroaryl-, Arylalkyl-, HeteroarylalkylRest steht,
R¹ und R² gleich oder verschieden sein können und für H, eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, eine Heteroatom-enthaltende Kohlenwasserstoffgruppe, die geeignete Substituenten haben kann, stehen und die Reste R¹ und R² einen Ring bilden können, der 4-bis 20-gliedrig, gesättigt oder ungesättigt, alicyclisch oder heteroalicyclisch sein kann und geeignete Substituenten aufweisen kann,
in welchem eine aromatische Verbindung mit der allgemeinen Formel II

Ar-X (II)

worin
Ar wie oben definiert ist und
X für F, Cl, Br, I oder OSO₂R³, in der R³ für eine Kohlenwasserstoffgruppe, wie eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, steht,
in Gegenwart eines Katalysators mit einem Amin der allgemeinen Formel III

H-NR¹R² (III)

worin R¹ und R² wie oben definiert sind,
und einer Base umgesetzt werden,
dadurch gekennzeichnet, dass der Katalysator ausgewählt ist aus Übergangsmetallkomplexen, die einen oder mehrere Liganden mit der allgemeine Formel IV 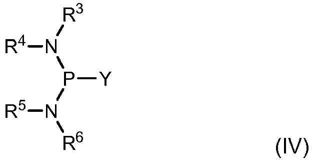 in der
R³, R⁴, R⁵ und R⁶ gleich oder verschieden sein können und für H, einen linearen oder verzweigten C₁-C₆-Alkylrest stehen, der ggf. substituiert sein kann, oder R³ und R⁴ und/oder R⁵ und R⁶ unter Bildung eines Ringes miteinander verbunden sind,
Y für Halogen oder einen Rest -OR⁷, worin R⁷ für H oder eine lineare oder verzweigte C₁-C₆Alkylkette, die ggf. substituiert sein kann, oder einen Arylrest, der ggf. substituiert sein kann, steht,
   aufweisen.

Die erfindungsgemäß verwendeten Phosphorverbindungen mit der allgemeinen Formel IV sind Verbindungen, die aus preiswerten Ausgangssubstanzen auf einfache Weise hergestellt werden können. Unter Einsatz dieser Katalysatoren ist es möglich, aromatische und heteroaromatische Amine kostengünstig herzustellen.

Die erfindungsgemäß verwendeten Katalysatoren sind Übergangsmetallkomplexverbindungen, die einen oder mehrere Liganden mit der allgemeinen Formel IV aufweisen.

Die Liganden mit der Formel IV stellen Derivate der phosphorigen Säure dar. Die Reste R³, R⁴, R⁵ und R⁶ können gleich oder verschieden sein und sind vorzugsweise ausgewählt aus linearen oder verzweigten Alkyl- und Alkenylgruppen mit 1 bis 6 Kohlenstoffatomen. Vorzugsweise stehen R³, R⁴, R⁵ und R⁶ für einen iso-Propyl, iso-Butyl, tert-Butyl, neoPentyl, und/oder tert-Amyl-Rest, wobei iso-Propyl und tert-Butyl besonders bevorzugt sind. In einer besonders bevorzugten Ausführungsform stehen R³ und R⁵ und/oder R⁴ und R⁶ für i-Propyl oder tert-Butyl.

Der Rest Y steht für Halogen, insbesondere Cl, Br oder I, oder einen Rest -OR⁷, worin R⁷ H, eine lineare oder verzweigte C₁-C₆-Alkylkette, die ggf. substituiert sein kann, oder einen Arylrest, der ggf. substituiert sein kann, bedeutet. Vorzugsweise ist Y ausgewählt aus Cl, Br oder -OR⁷, wobei R⁷ vorzugsweise i-Propyl oder tert-Butyl bedeutet.

Der im Rahmen der Erfindung verwendete Begriff Kohlenwasserstoffgruppe bedeutet eine gesättigte oder ungesättigte, verzweigte oder lineare Alkylgruppe, Alkenylgruppe, Alkinylgruppe, Arylgruppe, die geeignete Substituenten, auch Heteroatomsubstituenten, haben kann, oder eine Heteroatom-enthaltende Kohlenwasserstoffgruppe.

Alkyl kann unverzweigt (linear) oder verzweigt sein und hat 1 bis 6 Kohlenstoffatome. Vorzugsweise ist Alkyl Methyl, Ethyl, Propyl, Isopropyl, Butyl, Isobutyl, sec-Butyl oder tert-Butyl, ebenso Pentyl, 1-Methylpropyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1-oder 2Ethylbutyl, 1-Ethyl-1-methylpropyl, 1-Ethyl-2-methylpropyl, 1,1,2- oder 1,2,2-Trimethylpropyl. Alkyl kann auch für halogenierte Alkylreste stehen, z.B. für Trifluormethyl, Pentafluroethyl oder 1,1,1-Trifluorethyl.

Cycloalkyl ist vorzugsweise Cyclopropyl, Cyclobutyl, Cyclopentyl, Cyclohexyl oder Cycloheptyl. Alkylen bedeutet, vorzugsweise Methylen, Ethylen, Propylen, Butylen, Pentylen oder Hexylen, aber auch verzweigtes Alkylen.

Alkenyl ist bevorzugt Vinyl.

Alkinyl ist bevorzugt C=CH.

Halogen ist F, Cl, Br oder I.

Alkoxy ist vorzugsweise Methoxy, Ethoxy, Propoxy oder Butoxy.

C₃-C₈-Heterocycloalkyl mit einem oder mehreren Heteroatomen ausgewählt aus N, O und S ist vorzugsweise 2,3-Dihydro-2-, -3-, -4- oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2-oder 3-pyrrolidinyl, Tetrahydro-1-, -2- oder -4-imidazolyl, 2,3-Dihydro-1-, -2-, -3-, -4- oder - 5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-piperidinyl, 2-, 3-oder 4-morpholinyl, Tetrahydro-2-, -3- oder -4-pyranyl, 1,4-Dioxanyl, 1,3-Dioxan-2-, -4-oder -5-yl, hexahydro-1-, -3- oder -4-pyridazinyl, Hexahydro-1-, -2-, -4- oder -5-pyrimidinyl, 1-, 2- oder 3-piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8-3,4-Dihydro-2H-benzo-1,4-oxazinyl.

Gegebenenfalls substituiert bedeutet unsubstituiert oder mono-, di-, tri-, tetra- oder pentasubstituiert.

Aryl ist vorzugsweise Phenyl, Naphthyl oder Diphenyl.

Arylalkyl ist vorzugsweise Benzyl.

Heteroaryl mit einem oder mehreren Heteroatomen ausgewählt aus N, O und S ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, außerdem bevorzugt 1,2,3-Triazol-1-, -4- oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Chinolinyl, 2-, 4-, 5-, 6-, 7- oder 8-Chinazolinyl, 5- oder 6-Chinoxalinyl, 2-, 3-, 5-, 6-, 7-oder 8-2H-Benzo-1,4-oxazinyl, außerdem bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Beispiele für Substituenten sind C₁-C₄-Alk(en)yl, Aryl, Heteroaryl, Halogen, wie F, Cl, Br, I, NO₂, NR⁸R⁹, in der R⁸ und R⁹ gleich oder verschieden sein können und für H oder eine C₁-C₆Alkylgruppe stehen, usw.

Als Vorstufen für die Übergangsmetall-Komplexe kommen insbesondere Verbindungen der Eisenmetalle in Betracht, wie die Salze des Eisens, Palladiums, Nickels, Kobalts, Platins, Rhodiums oder Rutheniums. Es können auch Verbindungen eingesetzt werden, in denen das Übergangsmetall in der Oxidationsstufe Null vorliegt. Besonders bevorzugte Übergangsmetalle sind Palladium und Eisen. Insbesondere sind dabei Pd-Salze wie z.B. Pd(OAc)₂ (Ac = Acetyl), Pd(acac)₂ (acac = Acetylacetonat), (allPdCl)₂ (all = allyl) oder PdCl₂ geeignet, vorzugsweise Pd(OAc)₂ oder PdCl₂, ferner Pd⁰-Komplexe wie z.B. Pd₂(dba)₃ und Pd(dba)₂ (dba = Dibenzylidenaceton). Im Falle von Eisen sind insbesondere Salze des Typs FeCl₂ oder FeCl₃ zu erwähnen.

Zur Durchführung des erfindungsgemäßen Verfahrens kann die Menge des eingesetzten Übergangsmetalls zwischen 0.01 und 15 Mol-% variieren, wobei das molare Verhältnis von Ligand zu Übergangsmetall zwischen 3 : 1 und 1 : 1 eingestellt werden kann, vorzugsweise um 2 : 1.

Erfindungsgemäß wird das Verfahren in Gegenwart einer Base durchgeführt. Als Basen kommen insbesondere Alkalialkoholate, Alkalihydroxide, Alkalicarbonate und -phosphate, Ammoniak sowie organische Basen in Betracht. Beispielsweise können Lithium-, Natrium-, Kalium- oder Caesium-Alkoholate (ROMetall), wobei R ein primärer, sekundärer oder tertiärer C₁-C₄-Alkylrest sein kann, insbesondere CH₃ONa, (CH₃)₃CONa, (CH₃)₃COK, (CH₃)₂(C₂H₅)CONa oder (CH₃)₂(C₂H₅)COK, vorzugweise tertiäre Alkoholate, verwendet werden. Auch LHMDS (Li(NSiMe₃)₂), NaOH, KOH, (n-C₄H₉)₄NOH, Na₂CO₃, K₂CO₃, Cs₂CO₃ sowie K₃PO₄ können als Base dienen. Beispiele für organische Basen sind C₁-C₆-Alkylamin, Di-(C₁-C₆-alkyl)amin, Benzylamin, Piperidin und/oder Morpholin. Auch beliebige Gemische der voranstehenden Basen können eingesetzt werden.

Die Reaktion kann in üblichen polaren oder unpolaren organischen und auch ionischen Lösungsmitteln durchgeführt werden, beispielsweise Toluol, THF, Dimethoxyethan (DME), Dimethylformamid (DMF), Dimethylsulfoxid (DMSO), *N*-Methyl-2-pyrrolidon (NMP) oder 1,4Dioxan und beliebige Gemische daraus. Die Reaktion kann auch in wässrigen Lösungen durchgeführt werden. Es ist auch möglich, die Aminkomponente selbst als Lösungsmittel einzusetzen. Dieses ist beispielsweise möglich, wenn als Aminkomponente Ammoniak, C₁-C₆-Alkylamin, Di-(C₁-C₆-alkyl)amin, Benzylamin, Piperidin, Morpholin oder beliebige Gemische daraus eingesetzt werden.

Die Reaktion kann bei Raumtemperatur bis zu einer Temperatur von 180°C, vorzugsweise von 70°C bis 120°C durchgeführt werden.

### Beispiele

### Beispiel 1 - Aminierung von substituierten Arylhalogeniden mit Morpholin in Gegenwart von (i-Pr₂N)₂P-R-Liganden

Typische Beispiele für das erfindungsgemäße Verfahren sind die Reaktionen von Arylchloriden und Arylbromiden mit Morpholin (s. nachfolgendes Schema):

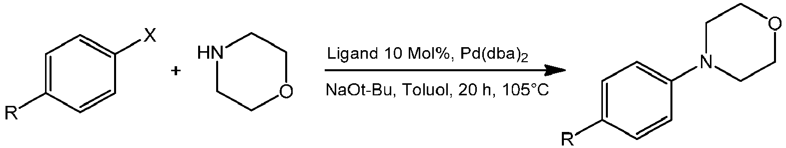

Zur Durchführung des Verfahrens wurden (25 mmol) Arylhalogenid und 3.13 g Morpholin (30 mmol) (2.62 ml) mit Toluol auf 25 ml aufgefüllt.

29 mg Pd(dba)₂ (0.05 mmol) und 125 mg Na-*Ot*-Bu (1.30 mmol) wurden in das Reaktionsgefäß eingewogen. Eine Lösung von 0.1 mmol ([(CH₃)₂CH]₂N)₂POC(CH₃)₃ in 2 ml Toluol wurde zum Pd(dba)₂ pipettiert und 10 min gerührt. Anschließend wurde 1 ml Substratlösung (1.0 mmol Arylhalogenid, 1.2 mmol Morpholin) zupipettiert, das erhaltene Gemisch wurde 20 h bei 105 °C gerührt.

Nach Beendigung der Reaktion ließ man das Reaktionsgemisch auf Raumtemperatur abkühlen, es wurden 50 ml Diethylether und 50 ml gesättigte NaCl-Lösung zugegeben. Die Wasserphase wurde 2mal mit 50ml Diethylether gewaschen, über MgSO₄ getrocknet, das Lösungsmittel wurde mittels Rotationsverdampfer entfernt. Das Reaktionsprodukt wurde aus dem Rückstand mittels Säulenchromatographie isoliert (Kieselgel 60, L = 11 cm, ∅ = 1.5cm, Pentan/Ether = 4:1).

Alternativ wurden nach dem Abkühlen auf Raumtemperatur 2.5 g Kieselgel 60 und 15 ml Diethylether zum Reaktionsgemisch zugegeben, 10 min gerührt, über einen Faltenfilter Kieselgel abfiltriert, mit Ether ausgiebig gewaschen, der Diethylether wurde mittels Rotationsverdampfer entfernt und der Rückstand im Vakuum getrocknet.

Die Reinheitsbestimmung der erhaltenen Produkte erfolgte mittels Gaschromatographie. Die Ergebnisse sind in Tabelle 1 dargestellt.

**Tabelle 1**

| S/C = 20; Pd/P = 1:2 | | | | |
|---|---|---|---|---|
| | 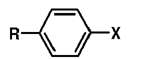 | **(i-Pr₂N)₂PCl** | **(i-Pr₂N)₂POt-Bu** | **(i-Pr₂N)₂POH** |
| | | Ausbeute (%) | Ausbeute (%) | Ausbeute (%) |
| **1** | R=H;X=Br | 74.8 | 100 | 43.9 |
| **2** | R = H; X = Cl | 92.2 | 86.0 | - |
| **3** | R = CN; X = Br | 31.3 | 47.5 | - |
| **4** | R = NO₂; X = Br | 36.8 | 71.0 | - |
| **5** | R = Me; X = Br | 84.0 | 88.0 | 53.3 |
| **6** | R = OMe; X = Br | 93.0 | 100 (s.u.) | - |
| **7** | R = Ph; X = Br | 72.7 | 76.3 | 45.2 |
| **8** | 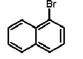 | 75.4 | 85.2 | 31.6 |
| **9** | R = Me; X = Cl | - | 92.4 | |
| **10** | R = *t-*Bu; X = Br | - | 92.9 | |
| **11** | R = *t*Bu; X = Cl | - | 94.6 | |
| **12** | R=F;X=Br | - | 90.3 | |
| **13** | R = Ph; X = Cl | - | 92.8 | |
| **14** | 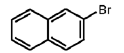 | - | 93.6 | |

### Beispiel 2 - Aminierung von Arylhalogeniden mit Piperidin und N-Methylbenzylamin in Gegenwart von (i-Pr₂N)₂P-R-Liganden

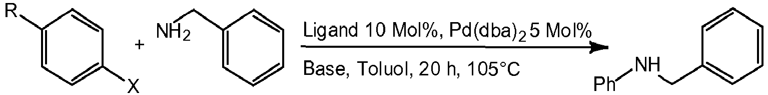

**Tabelle 2**

| S/C = 20; Pd/P = 1:2 | | | |
|---|---|---|---|
| | **Ligand** | **Amin/ArX** | **Ausbeute (%)** |
| | | | **Produkt** |
| **1** | (i-Pr₂N)₂PCl | Piperidin/PhBr | 84.6 |
| **2** | (i-Pr₂N)₂POt-Bu | Piperidin/PhBr | 73.9 |
| **3** | (i-Pr₂N)₂PCl | Piperidin/PhCl | 90.2 |
| **4** | (i-Pr₂N)₂POt-Bu | Piperidin/PhCl | 79.3 |
| **5** | (i-Pr₂N)₂PCl | Me(H)NBn/PhBr | 83.6 |
| **6** | (i-Pr₂N)₂POt-Bu | Me(H)NBn/PhBr | 83.3 |
| **7** | (i-Pr₂N)₂PCl | Me(H)NBn/PhCl | 78.8 |
| **8** | (i-Pr₂N)₂POt-Bu | Me(H)NBn/PhCl | 77.8 |

### Beispiel 3 - Aminierung von Arylhalogeniden mit Benzylamin in Gegenwart von (i-Pr₂N)₂P-Ot-Bu-Ligand

**Tabelle 3**

| S/C = 20; Pd/P = 1:2 | | |
|---|---|---|
| | 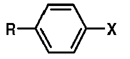 | **(i-Pr₂N)₂POt-Bu** |
| | | Ausbeute (%) |
| **1** | R = H;X = Br | 74.4 |
| **2** | R = H; X = Cl | 70.0 |
| **3** | R = CN; X = Cl | 51.4 |
| **4** | R = Me; X = Br | 70.0 |
| **5** | R = OMe; X = Br | 58.7 |
| **6** | R = Ph; X = Br | 88.4 |
| **7** | 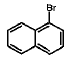 | 90.0 |

### Beispiel 4 - Aminierung von Arylbromid mit Morpholin in Gegenwart verschiedener Phosphor-Liganden

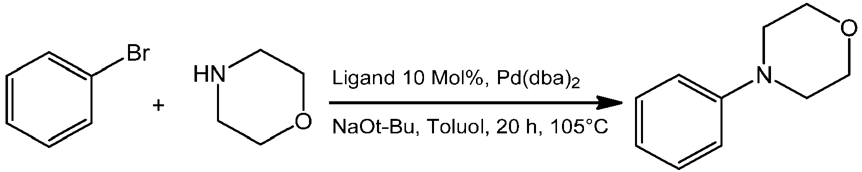

**Tabelle 4**

| | S/C = 20; Pd/P = 1:2 | |
|---|---|---|
| | **Ligand = (i-Pr₂N)₂P-R** | **Ausbeute (%)** |
| **1** | R = -CI | 74.8 |
| **2** | R = -OH | 87.2 |
| **3** | R = -O*t*-Bu | 100 |
| **4** | R = -O-adamantyl | 86.0 |
| **5** | R = -Benzyl * | 86.7 |
| **6** | R = -NMe₂ * | 82.8 |

| | **Ligand =** (O*t*-**Bu)₂P-NR₂** | |
|---|---|---|
| **7** | R = -Et * | 88.2 |
| **8** | R = -*i*-Pr * | 89.3 |

| | | |
|---|---|---|
| * Vergleichsbeispiele | | |

### Beispiel 5 - Aminierung von Bromheteroarenen mit Morpholin in Gegenwart von (i-Pr₂N)₂P-Ot-Bu-Ligand

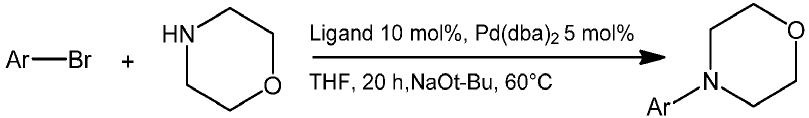

**Tabelle 5**

| S/C = 20; Pd/P = 1:2 | | |
|---|---|---|
| | Ar-Br | Ausbeute (%) |
| **1** | 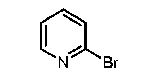 | 96.6 |
| **2** | 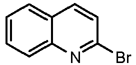 | 99.9 |
| **3** | 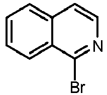 | 99.9 |

## Patentansprüche

1. Verfahren zur Herstellung von aromatischen Aminen der allgemeinen Formel (I)
Ar-NR¹R² (I)
worin
Ar für einen substitutierten oder unsubstituierten Aryl-, Heteroaryl-, Arylalkyl-, Heteroarylalkyl-Rest steht,
R¹ und R² gleich oder verschieden sein können und für H, eine Kohlenwasserstoffgruppe, wie eine gesättigte oder ungesättigte, verzweigte oder lineare Alkylgruppe, Alkenylgruppe, Alkinylgruppe, Arylgruppe, die Substituenten, auch Heteroatomsubstituenten, haben kann, eine Heteroatom-enthaltende Kohlenwasserstoffgruppe, die Substituenten haben kann, stehen und die Reste R¹ und R² einen Ring bilden können, der 4-bis 20-gliedrig, gesättigt oder ungesättigt, alicyclisch oder heteroalicyclisch sein kann und Substituenten haben kann,
in welchem eine aromatische Verbindung mit der allgemeinen Formel II
Ar-X (II)
worin
Ar wie oben definiert ist und
X für F, Cl, Br, I oder OSO₂R³, in der R³ für eine Kohlenwasserstoffgruppe, wie eine gesättigte oder ungesättigte, verzweigte oder lineare Alkylgruppe, Alkenylgruppe, Alkinylgruppe, Arylgruppe, die Substituenten, auch Heteroatomsubstituenten, haben kann, steht,
in Gegenwart eines Katalysators mit einem Amin der allgemeinen Formel III
H-NR¹R² (III)
worin R¹ und R² wie oben definiert sind,
und einer Base umgesetzt werden,
**dadurch gekennzeichnet, dass** der Katalysator ausgewählt ist aus Übergangsmetallkomplexen, die einen oder mehrere Liganden mit der allgemeinen Formel IV 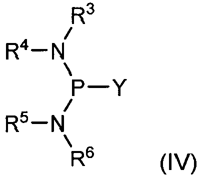 in der
R³, R⁴, R⁵ und R⁶ gleich oder verschieden sein können und für H, einen linearen oder verzweigten C₁-C₆-Alkylrest stehen, der ggf. substituiert sein kann, oder R³ und R⁴ und/oder R⁵ und R⁶ unter Bildung eines Ringes miteinander verbunden sind,
Y für Halogen oder einen Rest -OR⁷, worin R⁷ für H oder eine lineare oder verzweigte C₁-C₆-Alkylkette, die ggf. substituiert sein kann, oder einen Arylrest, der ggf. substituiert sein kann, steht,
aufweisen.

2. Verfahren nach Anspruch 1, **dadurch gekennzeichnet, dass** Y für Cl, Br oder -OR⁷ steht, wobei R⁷ i-Propyl oder tert-Butyl bedeutet.

3. Verfahren nach Anspruch 1 oder 2, **dadurch gekennzeichnet, dass** R³ und R⁵ und/oder R⁴ und R⁶ für i-Propyl oder tert-Butyl stehen.

4. Verfahren nach einem der Ansprüche 1 bis 3, **dadurch gekennzeichnet, dass** die Base ausgewählt ist aus Alkalihydroxiden, Alkalialkoholaten, Alkalicarbonaten und/oder Alkaliphosphaten, Ammoniak, C₁-C₆-Alkylamin, Di-(C₁-C₆-alkyl)amin, Benzylamin, Piperidin und/oder Morpholin.

5. Verfahren nach einem der Ansprüche 1 bis 4, **dadurch gekennzeichnet, dass** das Übergangsmetall ausgewählt ist aus Eisen, Palladium, Nickel, Kobalt, Platin, Rhodium und/oder Ruthenium.

6. Verfahren nach Anspruch 5, **dadurch gekennzeichnet, dass** zur Herstellung des Übergangsmetallkomplexes Übergangsmetallverbindungen ausgewählt aus Pd(OAc)₂, Pd(acac)₂, (allPdCl)₂, PdCl₂, Pd₂(dba)₃ und/oder Pd(dba)₂ verwendet werden.

## Claims

1. A process for preparing aromatic amines of the general formula (I)
Ar-NR¹R² (I)
wherein
Ar is a substituted or unsubstituted aryl, heteroaryl, arylalkyl, heteroarylalkyl radical,
R¹ and R² can be identical or different and are each H, a hydrocarbon group, such as a saturated or unsaturated, branched or linear alkyl group, alkenyl group, alkynyl group, aryl group, which may have suitable substituents, including heteroatom substituents, a heteroatom-containing hydrocarbon group which may have suitable substituents, and the radicals R¹ and R² may form a ring which can be 4- to 20-membered, saturated or unsaturated, alicyclic or heteroalicyclic and may have suitable substituents,
in which an aromatic compound having the general formula II
Ar-X (II)
where
Ar is as defined above and
X is F, Cl, Br, I or OSO₂R³ in which R³ is a hydrocarbon group, such as a saturated or unsaturated, branched or linear alkyl group, alkenyl group, alkynyl group, aryl group, which may have suitable substituents, including heteroatom substituents,
is reacted in the presence of a catalyst with an amine of the general formula III
H-NR¹R² (III)
where R¹ and R² are as defined above, and a base,
**characterized in that** the catalyst is selected from among transition metal complexes which have one or more ligands having the general formula IV 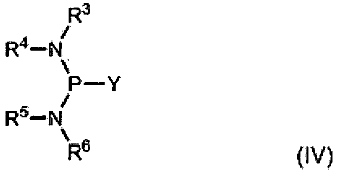 where
R³, R⁴, R⁵ and R⁶ can be identical or different and are each H, a linear or branched C₁-C₆-alkyl radical which may optionally be substituted, or R³ and R⁴ and/or R⁵ and R⁶ are joined to one another to form a ring,
Y is halogen or an -OR⁷ radical, where R⁷ is H or a linear or branched C₁-C₆-alkyl chain which may optionally be substituted or an aryl radical which may optionally be substituted.

2. The process as claimed in claim 1, **characterized in that** Y is Cl, Br or -OR⁷, where R⁷ is i-propyl or tert-butyl.

3. The process as claimed in claim 1 or 2, **characterized in that** R³ and R⁵ and/or R⁴ and R⁶ are i-propyl or tert-butyl.

4. The process as claimed in any of claims 1 to 3, **characterized in that** the base is selected from among alkali metal hydroxides, alkali metal alkoxides, alkali metal carbonates and/or alkali metal phosphates, ammonia, C₁-C₆-alkylamine, di(C₁-C₆-alkyl)amine, benzylamine, piperidine and/or morpholine.

5. The process as claimed in any of claims 1 to 4, **characterized in that** the transition metal is selected from among iron, palladium, nickel, cobalt, platinum, rhodium and ruthenium.

6. The process as claimed in claim 5, **characterized in that** transition metal compounds selected from among Pd(OAc)₂, Pd(acac)₂, (allPdCl)₂, PdCl₂, Pd₂(dba)₃ and Pd(dba)₂ are used for preparing the transition metal complex.

## Revendications

1. Procédé destiné à préparer des amines aromatiques de la formule générale (I)
Ar-NR¹R² (I)
dans laquelle
Ar est un radical aryle, hétéroaryle, arylalkyle, hétéroarylalkyle substitué ou non substitué,
R¹ et R² peuvent être identiques ou différents et signifient H, un groupe hydrocarbure, comme un groupe alkyle, un groupe alkényle, un groupe alkinyle, un groupe aryle saturé ou insaturé, ramifié ou linéaire, qui peut avoir des substituants, également des substituants hétéroatomiques, un groupe hydrocarbure contenant un hétéroatome, qui peut avoir des substituants et les radicaux R¹ et R² peuvent former un cycle, qui peut être à de 4 à 20 chaînons, peut être saturé ou insaturé, alicyclique ou hétéro alicyclique et peut avoir des substituants,
lors duquel on transforme un composé aromatique de la formule générale Il
Ar-X (II)
dans laquelle
Ar est défini comme ci-dessus
X signifie F, Cl, Br, I ou OSO₂R³ où R³ est un groupe hydrocarbure, comme un groupe alkyle, un groupe alkényle, un groupe alkinyle, un groupe aryle saturé ou insaturé, ramifié ou linéaire, qui peut avoir des substituants, également des substituants hétéroatomiques,
en présence d'un catalyseur avec un amine de la formule générale III
H-NR¹R² (III)
dans laquelle R¹ et R² sont définis comme ci-dessus,
et avec une base,
**caractérisé en ce que** le catalyseur est choisi parmi des complexes métalliques de transition, qui comportent un ou plusieurs ligands de la formule générale IV 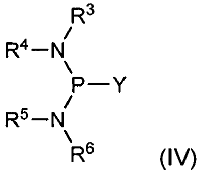 dans laquelle
R³, R⁴, R⁵ et R⁶ peuvent être identiques ou différents et signifient H, un radical alkyle en C₁ à C₆, linéaire ou ramifié, qui peut être substitué le cas échéant ou R³ et R⁴ et/ou R⁵ et R⁶ sont reliés entre eux, en formant un cycle,
Y signifie un halogène ou un radical -OR⁷ où R⁷ signifie H ou une chaine alkyle en C₁ à C₆, linéaire ou ramifiée, qui peut être substituée le cas échéant ou un radical aryle qui peut être substitué le cas échéant.

2. Procédé selon la revendication 1, **caractérisé en ce que** Y signifie Cl, Br ou OR⁷, R⁷ étant de l'i-propyle ou du tert-butyle.

3. Procédé selon la revendication 1 ou la revendication 2, **caractérisé en ce que** R³ et R⁵ et/ou R⁴ et R⁶ sont de l'i-propyle ou du tert-butyle.

4. Procédé selon l'une quelconque des revendications 1 à 3, **caractérisé en ce que** la base est choisie parmi les hydroxydes alcalins, les alcoolates alcalins, les carbonates alcalins et/ou les phosphates alcalins, l'ammoniac, un alkylamine en C₁ à C₆, un di-(C₁-C₆-alkyl)amine, un benzylamine, une pipéridine et/ou une morpholine.

5. Procédé selon l'une quelconque des revendications 1 à 4, **caractérisé en ce que** le métal de transition est choisi parmi le fer, le palladium, le nickel, le cobalt, le platine, le rhodium et/ou le ruthénium.

6. Procédé selon la revendication 5, **caractérisé en ce que** pour produire le complexe métallique de transition, on utilise des composés métalliques de transition, choisis parmi le Pd(OAc)₂, le Pd(acac)₂, l'(aIIPdCl)₂, le PdCl₂, le Pd₂(dba)₃ et/ou le Pd(dba)₂.
